Europäisches Patentamt

European Patent Office

Office européen des brevets

(11) Numéro de publication: **0 126 690**
**A1**

## DEMANDE DE BREVET EUROPEEN

(21) Numéro de dépôt: 84401010.8

(22) Date de dépôt: 17.05.84

(51) Int. Cl.³: **H 04 R 1/10, A 61 F 11/02**

(30) Priorité: 19.05.83 FR 8308273

(43) Date de publication de la demande: 28.11.84
Bulletin 84/48

(84) Etats contractants désignés: **DE GB IT**

(71) Demandeur: **FAME, La Fontaine St Remy de la Vanne,
F-77320 La Ferte Gaucher (FR)**

(72) Inventeur: **Tabardel, Christian, 91, rue Jules Guesde,
F-94490 Ormesson (FR)**
Inventeur: **Piat, Jean-Claude, 4 le Petit Barlonges,
F-77320 St Remy de la Vanne (FR)**
Inventeur: **Giraud, Jean-Pierre, Montmogis, F-77320 St
Remy de la Vanne (FR)**
Inventeur: **Pernin, Henry, 14 Avenue des Alliés,
F-77320 La Ferte Gaucher (FR)**

(74) Mandataire: **Tony-Durand, Serge, Cabinet
Tony-Durand 22, Boulevard Voltaire, F-75011 Paris (FR)**

(54) **Casque de protection et dispositif émetteur de signaux sonores.**

(57) Casque de protection contre un environnement hostile ou gênant, comprenant deux oreillettes portées par un arceau élastique.

Ce casque comporte un dispositif (11) émetteur de signaux sonores, intégré dans l'une de ces oreillettes (2), et un appareillage de commande également intégré dans l'une ou l'autre de ces oreillettes. Celui-ci comprend un programmateur (12) associé à un cadran de commande (9) susceptible d'être actionné par l'utilisateur pour déterminer la programmation de fonctionnement du dispositif (11) émetteur de signaux sonores.

Ce casque de protection peut être utilisé dans tous les cas où il convient de transmettre des signaux sonores, par exemple d'indication de temps, à une personne se trouvant placée dans un environnement hostile ou gênant.

"Casque de protection et dispositif émetteur de signaux sonores"

La présente invention concerne les casques de protection contre les nuisances extérieures, qui sont destinés à être utilisés dans un environnement hostile ou gênant.

Les casques actuels de ce genre sont en général constitués par deux oreillettes destinées à être appliquées contre les oreilles de l'utilisateur et qui sont portées par un arceau élastique. Ces casques assurent purement et simplement une protection de l'usager, par exemple contre des bruits trop importants à l'intérieur d'un stand de tir ou sur un banc d'essai de réacteur etc...

Cependant dans certaines circonstances il est nécessaire de pouvoir transmettre, à l'utilisateur d'un tel casque, des signaux d'avertissement, par exemple pour lui signaler certaines limites de temps à respecter ou toutes autres informations utiles. Toutefois ceci se révèle impossible du fait même qu'un tel casque empêche son utilisateur d'entendre de tels signaux.

C'est pourquoi la présente invention a pour but de réaliser un casque de protection conçu de façon à assurer la transmission de signaux sonores selon tout programme voulu, ce casque étant agencé de manière à être entièrement autonome.

A cet effet il est caractérisé en ce qu'il comporte un dispositif émetteur de signaux sonores intégré dans l'une de ses oreillettes et un appareillage de commande également intégré dans l'une ou l'autre de ses oreillettes et qui comprend un programmateur associé à un cadran de commande susceptible d'être actionné par l'utilisateur pour déterminer la programmation de fonctionnement du dispositif émetteur de signaux sonores.

Un tel casque de protection peut faire l'objet de diverses applications dont certaines seront précisées par la suite.

Un exemple de réalisation de ce casque est décrit ci-dessous en référence au dessin annexé à simple titre indicatif et sur lequel :

La figure 1 est une vue en coupe-élévation d'un casque de protection selon l'invention ;

Les figures 2 et 3 sont des vues en élévation de l'une et l'autre des deux oreillettes de ce casque ;

La figure 4 est une vue du schéma électrique du dispositif intégré dans les oreillettes de ce casque.

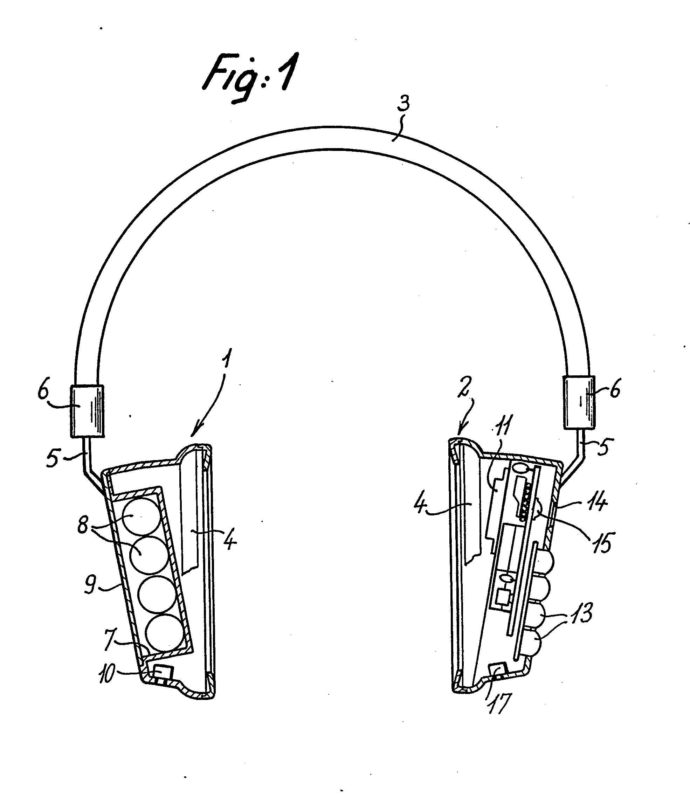

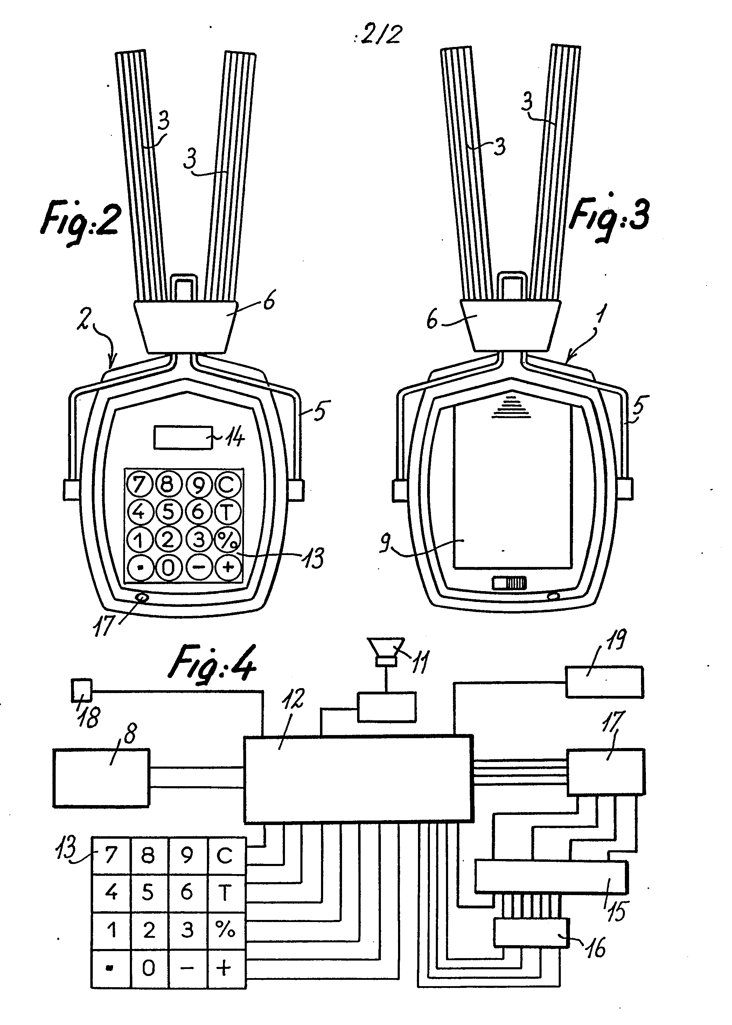

Le casque de protection représenté aux figures 1 à 3 comporte deux oreillettes, désignées par les références générales 1 et 2, qui sont destinées à être appliquées contre l'une et l'autre oreilles de l'utilisateur. Ces oreillettes sont portées par un double arceau élastique 3 susceptible d'être placé à cheval sur la tête de celui-ci. Chaque oreillette est constituée par un petit boîtier fabriqué en une matière plastique moulée, ou autre matière appropriée, et qui est pourvu d'un coussin souple permettant une adaptation aisée sur les oreilles de l'utilisateur dans des conditions confortables d'emploi.

Ces oreillettes comportent une garniture 4 en matériau insonorisant de façon à assurer l'isolation phonique voulue. A ce sujet il convient de noter que le coussin prévu sur chaque oreillette a également pour fonction de réaliser une isolation de l'oreille correspondante par rapport aux bruits extérieurs.

Chaque oreillette est montée de façon pivotante sur un étrier 5 lui-même monté coulissant dans un embout 6 porté par l'extrémité correspondante de l'arceau 3, des moyens appropriés étant prévus pour assurer une immobilisation dans la position de réglage voulue. Ceci permet une très bonne adaptation de la position des oreillettes en fonction de la morphologie de l'utilisateur. Quant à l'arceau 3 il est fabriqué en matériaux souples et élastiques permettant de s'adapter à la tête de l'utilisateur, tout en assurant une application élastique des oreillettes contre les oreilles de celui-ci. Par ailleurs cet arceau renferme ou porte des conducteurs électriques assurant la liaison entre les deux parties du dispositif intégré à l'intérieur des deux oreillettes 1 et 2.

Dans l'exemple représenté, l'une de ces oreillettes, en l'occurrence l'oreillette 1 comporte un compartiment 7 destiné à servir de logement à des piles 8 alimentant le circuit électrique du dispositif intégré à ce casque. De préférence il s'agit de piles au cadmium-nickel qui sont rechargeables sur le secteur électrique d'alimentation et ce, grâce à une prise 10 prévue dans l'oreillette correspondante 1 et dans laquelle peut être branchée une fiche portée par un conducteur de raccordement.

Quant à l'autre oreillette,elle comporte un dispositif couineur ou ronfleur 11 placé en regard d'ouvertures prévues sur la face interne de cette oreillette de façon que les sons émis par ce dispositif soient directement transmis à l'oreille correspondante de l'utilisateur.

Ce dispositif émetteur de sons est commandé par un programmateur 12 qui assure son alimentation aux moments voulus. Suivant les cas et applications, ce programmateur peut comporter une ou plusieurs mémoires statiques ainsi qu'une ou plusieurs mémoires modifiables pouvant être programmées au moyen d'un clavier de commande 13 comportant une série de touches de manoeuvre. Ce clavier est disposé sur la face externe de l'oreillette correspondante 2 pour pouvoir être manoeuvré aisément. Sur cette même face est prévu un voyant 14 derrière lequel est disposé un système afficheur 15 permettant de contrôler les données ainsi programmées. Ce système afficheur est relié au programmateur 12,à la fois par un dispositif 16 de décodage, et par un système 17 de sélection des affichages désirés.

Ce casque de protection est susceptible d'être utilisé, par exemple, par des personnes se trouvant dans un milieu sonore important auxquelles des informations de temps doivent être signalées d'une manière précise, répétitive ou non, en fonction par exemple d'un programme déterminé.

4

Ainsi un tel casque peut être utilisé par des tireurs pour respecter un certain programme déterminé. Dans un tel cas le programmateur 12 peut être réglé pour que le couineur 11 émette des tops sonores à des moments précis correspondant à une cadence déterminée de tir. Ainsi un tireur peut s'entraîner successivement à des cadences de tir de plus en plus rapides pour être ultérieurement en mesure de respecter une cadence imposée au cours d'un concours ou d'une épreuve sportive.

Cependant le casque de protection selon l'invention peut faire l'objet de nombreuses autres applications pour lesquelles il existe des problèmes d'ordre similaire. Ainsi il peut être utilisé par des travailleurs effectuant des travaux dans une atmosphère dangereuse où la durée de présence doit être limitée. Dans un tel cas il est possible de programmer chaque période de présence en fonction des mesures de sécurité à respecter. Mais, sous réserve d'être rendu étanche, ce casque peut également être employé par des plongeurs devant respecter certaines mesures de sécurité, par exemple des paliers de décompression pendant leur remontée. En effet les durées à respecter pour chaque palier peuvent alors être programmées et ensuite transmises à l'utilisateur grâce aux tops sonores émis par le couineur 11.

Mais encore une fois, le casque selon l'invention peut faire l'objet de nombreuses autres applications.

Eventuellement l'oreillette de commande 2 peut comporter une prise 18 permettant son raccordement avec un dispositif de télécommande. Il peut également être prévu une ou plusieurs autres prises 19 permettant de brancher des conducteurs aboutissant à des sorties auxiliaires, par exemple des unités périphériques devant être commandées par le programmateur 12.

Néanmoins il s'agit là de simples possibilités supplémentaires qui ne sont en aucune façon indispensables. En effet le principal avantage du casque selon l'invention est son autonomie complète puisque, selon la caractéristique essentielle de celui-ci, le dispositif émetteur de sons et son système de commande sont intégrés à l'intérieur de l'une des deux oreillettes cependant que les prises d'alimentation sont intégrées dans l'autre. Ceci autorise du reste une liberté totale de mouvement de l'utilisateur permettant l'emploi de ce casque dans des circonstances où il serait gênant, ou même impossible, que l'utilisateur soit relié par un conducteur souple à un dispositif de commande quelconque.

6

## REVENDICATIONS

1. Casque de protection contre un environnement hostile ou gênant, comprenant deux oreillettes portées par un arceau élastique, caractérisé en ce qu'il comporte un dispositif (11) émetteur de signaux sonores, intégré dans l'une de ces oreillettes (2) et un appareillage de commande également intégré dans l'une ou l'autre de ces oreillettes et qui comprend un programmateur (12) associé à un cadran de commande (9) susceptible d'être actionné par l'utilisateur pour déterminer la programmation de fonctionnement du dispositif (11) émetteur de signaux sonores.

2. Casque de protection selon la revendication 1, caractérisé en ce que le cadran (9) de commande du programmateur (12) est disposé sur la face externe de l'oreillette (2) renfermant celui-ci ainsi que le dispositif (11) émetteur de sons.

3. Casque de protection selon l'une des revendications précédentes, caractérisé en ce que, l'une des oreillettes (2) contenant le dispositif (11) émetteur de signaux sonores ainsi que tout l'appareillage de commande associé à ce dispositif, l'autre oreillette (1) comporte un compartiment (7) servant de logement à des piles d'alimentation de ce dispositif et de cet appareillage, et des conducteurs de liaison sont portés par l'arceau (3) sur lequel sont fixées ces oreillettes.

4. Casque de protection selon l'une des revendications précédentes, caractérisé en ce que l'oreillette (2), renfermant le dispositif (11) émetteur de sons et son appareillage de commande, comporte une prise (17) permettant de brancher un conducteur de raccordement à des appareils extérieurs devant être commandés par le programmateur.

Fig.1

Fig:2

Fig:3

Fig:4

Office européen
des brevets

**RAPPORT DE RECHERCHE EUROPEENNE**

Numéro de la demande

EP  84 40 1010

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | CH-A- 487 639 (RUEPP) <br> * Colonne 2, ligne 31 - colonne 3, ligne 34; figure * | 1-4 | H 04 R 1/10 <br> A 61 F 11/02 |
| A | US-A-4 301 524 (KOEPP et al.) <br> * Résumé; figure 2 * | 1 | |
| A | FR-A-1 512 289 (KAHN) <br> * Page 5, colonne de gauche, dernière ligne - colonne de droite, ligne 15; figure 2 * | 1,2 | |
| A | FR-A-2 014 846 (AMPLIVOX) <br> * Page 4, lignes 13-15; figure 1 * | 1 | |
| A | DE-A-1 809 354 (CHARLTON) <br> * Page 5, ligne 31 - page 6, ligne 3; figure 3 * | 1,4 | DOMAINES TECHNIQUES RECHERCHES (Int. Cl. ³) |
| A | FR-A-2 515 456 (LEM) <br> * Page 1, ligne 37 - page 2, ligne 7; figure 1 * | 1,2,4 | H 04 R <br> A 61 F <br> G 04 G <br> G 04 F |
| A | DE-A-2 132 817 (A.K.G.) <br> * Page 6, lignes 8-16 * | 1,2 | |

Le présent rapport de recherche a été établi pour toutes les revendications

| Lieu de la recherche | Date d'achèvement de la recherche | Examinateur |
|---|---|---|
| LA HAYE | 13-08-1984 | LUBERICHS A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82